# FASCICULE DE BREVET EUROPEEN

(11) **EP 4 551 155 B1**
(45) Date de publication et mention de la délivrance du brevet: **18.02.2026**
(21) Numéro de dépôt: 23736143.1
(22) Date de dépôt: 30.06.2023
(51) Int. Cl.: A61F 2/06

(54) **SYSTEME D'ANASTOMOSE**
ANASTOMOSESYSTEM
ANASTOMOSIS SYSTEM

(30) Priorité: 04.07.2022 FR 2206763
(43) Date de publication de la demande: 14.05.2025
(73) Titulaire: Assistance Publique - Hôpitaux de Paris, 75012 Paris (FR)
(72) Inventeur: DANIAL, Pichoy, 94700 Maisons-Alfort (FR)
(74) Mandataire: Cabinet Camus Lebkiri
(86) Numéro de dépôt international: PCT/EP2023/068106
(87) Numéro de publication internationale: WO 2024/008592

(56) Documents cités:
- WO-A1-2011/003019
- WO-A1-2018/211242
- WO-A2-2014/028787
- CN-A- 106 726 001

## Description

### DOMAINE TECHNIQUE DE L'INVENTION

Le domaine technique de l'invention se rapporte au domaine des systèmes d'anastomose vasculaire utilisés dans la chirurgie de revascularisation.

La présente invention concerne un système d'anastomose.

L'invention trouve une application dans le traitement de patients souffrant de maladie vasculaire, ainsi que dans l'utilisation de pompe de déchargement cardiaque.

### ARRIERE-PLAN TECHNOLOGIQUE DE L'INVENTION

De façon générale, une anastomose chirurgicale est une connexion artificielle faite par un chirurgien entre deux portions d'un même vaisseau, ou entre deux portions de deux vaisseaux, ou entre deux organes.

L'anastomose chirurgicale vasculaire est en particulier utilisée pour réparer les artères et les veines blessées ou endommagées.

Elle est ainsi souvent indiquée dans les pathologies de l'aorte : anévrisme, artéropathie oblitérante des membres inférieurs, lésions aorto-occlusives, etc. Dans ces pathologies, une partie du vaisseau est obstruée ou altérée. L'anastomose consiste alors à rediriger le flux de sang en dehors de cette partie via une prothèse, par exemple un tube synthétique, ce tube communiquant avec le vaisseau de part et d'autre de la partie obstruée.

Dans cette configuration, l'étape d'anastomose dans un vaisseau désigne l'insertion de l'extrémité de la prothèse dans le vaisseau, pour la connexion de cette prothèse avec le volume de cette partie du vaisseau.

De façon générale, un système d'anastomose fait référence à une prothèse et aux dispositifs interventionnels qui y sont associés.

Parmi les systèmes d'anastomose vasculaire, on distingue deux types de systèmes selon leur prise en charge : on trouve ainsi les systèmes dits « totalement endovasculaires » pris en charge par voie totalement endovasculaire, et les prothèses vasculaires prises en charge par chirurgie ouverte conventionnelle.

Les systèmes pris en charge par voie totalement endovasculaire ne nécessitent pas de chirurgie ouverte ni de laparatomie. Ils sont introduits dans les vaisseaux par ponction à travers la peau ou par un très court abord chirurgical. Ils sont généralement désignés sous le nom d'endoprothèse ou endoanastomose. Théoriquement, ils entrainent une diminution de la morbi-mortalité. Ils ont cependant encore un taux de complications péri-opératoires et de ré-opérations non négligeables.

Les systèmes d'anastomose pris en charge par la voie chirurgicale conventionnelle ouverte restent ainsi la référence. Ils consistent à aborder l'anastomose par l'extérieur du vaisseau.

Le problème rencontré est que ces systèmes nécessitent une interruption temporaire de la circulation du sang dans le vaisseau, le temps de l'étape d'anastomose dans les régions exempte de maladie du vaisseau. Cette interruption temporaire de la circulation sanguine, appelée aussi clampage, a des effets négatifs pour le patient, rendant le traitement inapplicable aux patients les plus fragiles, parmi lesquels les personnes âgées ou les personnes à haut risque.

Le risque de complications est particulièrement important lorsque le clampage est supra-rénal, comme c'est le cas dans le traitement d'artériopathie oblitérante des membres inférieurs avec des lésions aorto-occlusives ou dans le traitement des anévrismes de l'aorte thoraco-abdominale ou abdominale étendue aux artères rénales, mésentériques supérieure ou coeliaque. En effet, le temps d'interruption est long, de l'ordre de 40-45 min, car il s'agit d'anastomoser la prothèse sur une grande longueur. Pendant ce temps, des organes importants comme les reins ou le foie ne sont pas irrigués et le cœur subit une pression particulière. Il en résulte un risque accru d'accident vasculaire cérébral, d'insuffisance rénale, d'ischémie digestive ou encore d'infarctus du myocarde.

Il existe donc un besoin pour des systèmes d'anastomose permettant d'établir une anastomose dans un vaisseau ou un organe sans interruption de la circulation de sang dans le vaisseau ou l'organe, tout en évitant toute perte de sang.

Ce besoin s'exprime aussi dans le cas spécifique des systèmes d'assistance circulatoire du cœur, en particulier dans le cas de systèmes de pompes à sang de déchargement tels que décrits par exemple dans la demande FR3112579A1. De tels systèmes bénéficieraient notamment d'un accès à l'oreillette gauche du cœur par anastomose évitant toute perte de sang.

WO2014028787 A2 divulgue des conduits intra-vasculaires améliorés présentant une résistance accrue à la thrombose ; des manchettes pour site d'insertion de conduit résistant aux infections ; et des orifices latéraux de conduit permettant l'insertion facile de dispositifs médicaux à base de fil-guide et de cathéter pour traiter les conduits et vaisseaux sanguins afférents, afin de maintenir le fonctionnement du système de pompe à sang au fil du temps.

### RESUME DE L'INVENTION

L'invention offre une solution aux problèmes évoqués précédemment, en proposant un système d'anastomose comportant des dispositifs anti-reflux limitant les pertes de sang et évitant le clampage.

Pour ce faire, un premier aspect de l'invention a pour objet un système d'anastomose remarquable en ce qu'il comporte :
- un premier tube présentant un axe longitudinal, une surface latérale, une ouverture amont et une ouverture aval ;
- au moins un deuxième tube s'étendant depuis la surface latérale du premier tube jusqu'à une entrée du deuxième tube et communiquant avec le premier tube en traversant de façon étanche la surface latérale, la zone de la surface latérale traversée par le deuxième tube définissant une zone de communication entre le premier et le deuxième tube ;
- une valve montée dans le deuxième tube ; et
- un dispositif de ligature agencé sur la circonférence du premier tube en aval de la zone de communication entre le premier tube et le deuxième tube, le dispositif de ligature présentant un premier agencement ou un deuxième agencement, le premier agencement fermant le premier tube et le deuxième agencement laissant ledit premier tube ouvert.

Le système selon l'invention comprend ainsi une prothèse (le premier tube) comportant une dérivation (le deuxième tube) qui permet de recevoir les instruments interventionnels nécessaires à la réalisation d'une anastomose dans un vaisseau ou un organe, cette anastomose étant alors réalisée au niveau de l'ouverture amont du premier tube.

Plus précisément, le système selon l'invention permet de recevoir des dispositifs médicaux utilisés en chirurgie endovasculaire, parmi lesquels un guide de franchissement adapté pour percer la paroi du vaisseau ou de l'organe et un guide de maintien adapté pour réaliser la jonction entre la prothèse et le volume du vaisseau ou de l'organe.

Grâce à l'invention, l'anastomose est réalisée sans perte de sang, de façon rapide, simple et reproductible, et en respectant au mieux la paroi des vaisseaux. En d'autres termes, l'invention permet de réaliser une anastomose mini-invasive et sans pertes de sang. Le système évite ainsi le recours au clampage du vaisseau en amont du site d'anastomose, ou alors ce clampage est réduit à une durée très courte, de l'ordre de quelques minutes, par exemple 5 minutes. Ainsi, le système peut être proposé à des patients âgés ou fragiles qui sont généralement contre-indiqués à la chirurgie classique.

Plus précisément, les pertes de sang sont évitées grâce à :
- une première étanchéité, assurée avant même le franchissement de la paroi du vaisseau, par simple suture de la prothèse autour de l'ouverture amont et de l'ouverture aval sur le vaisseau ou l'organe,
- une deuxième étanchéité, assurée par la valve et le dispositif de ligature configuré dans son premier agencement, ceux-ci évitant tout reflux de sang hors du système lorsque la paroi du vaisseau ou de l'organe est franchie, c'est-à-dire percée, par le guide de franchissement,
- une troisième étanchéité, assurée par le guide de maintien au niveau de la jonction entre le vaisseau ou l'organe et le système, le guide de maintien permettant de réaliser une endo-anastomose.

Le dispositif de ligature est un clip de ligature agencé sur la circonférence du premier tube, le clip de ligature étant muni d'une vis de ligature.

Ainsi, il est possible d'actionner à distance et de façon rapide et simple le serrage et le desserrage du clip via la vis de ligature, pour configurer ce clip, respectivement, dans le premier agencement et le deuxième agencement.

Avantageusement, le système peut comprendre un fil-guide support s'étendant de l'entrée du deuxième tube à l'ouverture amont du premier tube en passant par la valve, le fil-guide support définissant un trajet de guidage.

Le fil-guide support permet de guider de façon simple et reproductible les instruments interventionnels à travers le système.

Avantageusement, le système peut comprendre en outre au moins un instrument interventionnel choisi parmi un ensemble d'instruments interventionnels comprenant un guide de franchissement adapté pour percer la paroi d'un vaisseau ou d'un organe et un guide de maintien adapté pour réaliser la jonction entre le premier tube et le volume du vaisseau ou de l'organe, chaque instrument interventionnel étant configuré pour suivre le trajet de guidage.

Le système permet ainsi de réaliser les étapes d'anastomose avec des instruments de type endovasculaires pour une anastomose mini-invasive.

Avantageusement, le premier tube présente un premier diamètre et le deuxième tube présente un deuxième diamètre, le premier diamètre pouvant être égal à 9F, 10 F, 12F, 14F, 16F ou 18F, et le deuxième diamètre pouvant être égal à 6F ou 8F.

Ainsi, le premier tube est compatible avec une prothèse vasculaire, et le deuxième tube est compatible avec le passage des instruments d'anastomose et avec l'aménagement, à l'intérieur de celui-ci, d'une valve.

Avantageusement, le guide de maintien coopère avec le fil-guide support et comprend un stent, le stent étant couvert et présentant une forme déployée de diamètre supérieur au diamètre de premier tube, la forme déployée prolongeant le premier tube à partir de l'extrémité amont du premier tube.

En d'autres termes, le stent est déployé depuis l'intérieur du premier tube au niveau de l'ouverture amont, de sorte que sa forme déployée se situe dans le prolongement du premier tube en étant adjacente à l'ouverture amont. Le stent réalise ainsi une jonction stable et étanche, et cela sur une longue durée, entre la prothèse et le vaisseau ou l'organe. Cela est particulièrement avantageux lorsque le système est appliqué sur un organe comme le cœur, car le maintien est stable et de bonne qualité malgré les mouvements liés à la contraction du cœur.

Par ailleurs, grâce au fil-guide support, le guide de maintien est positionné de façon simple, reproductible et rapide.

Un avantage d'un stent couvert est qu'il permet d'assurer l'étanchéité de la jonction entre la prothèse et le vaisseau, évitant ainsi toute perte de sang.

Avantageusement, le stent peut être un stent couvert sur ballon.

Le ballon permet de déployer le stent.

Dans un premier mode de réalisation, le premier tube est un tube simple, sans bifurcation (ou ramification ou dérivation).

Selon un développement de ce premier mode de réalisation, un troisième tube traverse le système en suivant le trajet de guidage, et le stent comprend en plus un système d'accroche.

Avantageusement, le système d'accroche peut être un autre stent.

Ce développement trouve un avantage particulier lorsqu'il est appliqué sur le septum inter-atrial du cœur d'un patient et utilisé avec un système d'assistance circulatoire pour décharger la partie gauche du cœur et réduire l'insuffisance cardiaque. En effet, le troisième tube permet de faire circuler le sang de l'oreillette gauche du cœur vers le système d'assistance en évitant un accès périphérique à travers le muscle cardiaque à l'oreillette gauche. L'avantage est que l'abord chirurgical est beaucoup moins invasif, une thoracotomie n'étant plus nécessaire.

Par ailleurs, le système d'accroche permet d'améliorer le maintien de l'anastomose notamment vis-à-vis des mouvements cardiaques.

Dans un deuxième mode de réalisation, le premier tube comporte une bifurcation en deux tubes, la bifurcation définissant sur le premier tube une partie droite et une partie bifurquée, la bifurcation étant agencée en aval du dispositif de ligature de sorte que la partie droite du premier tube se trouve en amont du système de ligature et la partie bifurquée se trouve en aval dudit dispositif de ligature.

La ramification de la prothèse en deux branches aval trouve un avantage particulier lors d'une anastomose réalisée sur l'aorte abdominale. En effet, en aval, la prothèse peut être anastomosée directement sur les deux artères iliaques via les deux bifurcations. L'anastomose est ainsi rapide, plus rapide que pour une prothèse classique où l'anastomose est termino-terminale, c'est-à-dire effectuée latéralement, sur toute la longueur de la prothèse, le long de l'aorte et de la prothèse. L'avantage apporté par le premier tube bifurqué est qu'il évite le recours à un clampage long, entrainant une intervention plus légère.

Selon un premier développement de ce deuxième mode de réalisation, la partie bifurquée du premier tube communique avec au moins un quatrième tube, le quatrième tube s'étendant entre la surface latérale de la partie bifurquée du premier tube et une entrée du quatrième tube.

Le quatrième tube constitue une ramification supplémentaire de la prothèse destinée à être anastomosée sur un vaisseau ou un organe. Un avantage particulier est trouvé lorsque le système est appliqué sur l'aorte abdominale. En effet, le quatrième tube peut être anastomosé sur une des artères principales (artères rénales et mésentériques) alimentées par l'aorte. Le troisième tube permet ainsi de réduire le temps nécessaire à la réalisation de l'anastomose.

Avantageusement, la partie bifurquée du premier tube peut communiquer avec un cinquième, un sixième et un septième tube, le cinquième, sixième et septième tubes étant espacés les uns des autres sur la surface latérale de la partie bifurquée du premier tube, l'espacement étant longitudinal ou circonférentiel.

Ainsi, les troisième, cinquième, sixième et septièmes tubes sont disposés de façon à être facilement anastomosés sur les les artères viscérales principales : artère rénale droite, artère rénale gauche, artère coeliaque et artère mésentérique supérieure.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La [Fig. 1] montre une représentation schématique du système selon un premier mode de réalisation de l'invention ;
- La [Fig. 2] montre une représentation schématique du système selon un premier mode de réalisation de l'invention, avec le dispositif de serrage en configuration serrée ;
- La [Fig. 3] montre un diagramme en bloc illustrant les étapes de mise en place du système de l'invention dans un vaisseau ;
- La [Fig. 4] montre une représentation schématique en coupe du système de l'invention lors de la deuxième étape de mise en place dans un vaisseau ;
- La [Fig. 5] montre une représentation schématique en coupe du système de l'invention lors de la troisième étape de mise en place dans un vaisseau ;
- La [Fig. 6] montre une représentation schématique en coupe du système de l'invention lors de la quatrième étape de mise en place dans un vaisseau ;
- La [Fig. 7] montre une représentation schématique d'un stent couvert sur ballon agencé sur l'extrémité d'un dispositif instrumental dans le système selon l'invention ;
- La [Fig. 8] montre une représentation schématique d'un stent auto-expansible agencé sur l'extrémité d'un dispositif instrumental dans le système selon l'invention ;
- La [Fig. 9] montre une représentation schématique en coupe du système mis en place de part et d'autre d'un segment pathologique d'un vaisseau ;
- La [Fig. 10] montre une représentation schématique en perspective du système mis en place de part et d'autre d'un segment pathologique d'un vaisseau ;
- La [Fig. 11] montre un système de pompe à sang de déchargement pour réduire l'insuffisance cardiaque relié à l'oreillette gauche d'un cœur par le système d'anastomose de l'invention ;
- La [Fig. 12] montre une représentation schématique du système selon un premier développement d'un deuxième mode de réalisation de l'invention ;
- La [Fig. 13] montre une représentation schématique du système selon un deuxième développement d'un deuxième mode de réalisation de l'invention ;
- La [Fig. 14] montre une représentation schématique en coupe du système selon un développement du premier mode de réalisation de l'invention, ledit système étant mis en place sur un vaisseau ou un organe ;
- La [Fig. 15] montre un système de pompe à sang de déchargement pour réduire l'insuffisance cardiaque relié à l'oreillette gauche d'un cœur par le septum inter-atrial avec le système selon le développement du premier mode de réalisation de l'invention.

### DESCRIPTION DETAILLEE

L'invention se rapporte à un système d'anastomose évitant les pertes de sang.

Le terme "anastomose", tel qu'utilisé ici, signifiera la connexion chirurgicale d'une prothèse synthétique, de structure tubulaire, dans une structure naturelle, celle-ci pouvant être un vaisseau ou un organe, pour la connexion de cette prothèse avec le volume de ce vaisseau ou de cet organe.

Nous discuterons principalement d'anastomose sur l'aorte et sur l'oreillette gauche du cœur. Naturellement, l'invention pourra être utilisée en variante pour la connexion d'autres structures naturelles.

Le terme « endo-anastomose » sera plus particulièrement utilisé lorsque l'anastomose est obtenue par l'intérieur de la structure naturelle.

Par ailleurs, les termes « amont » et « aval » feront référence à la circulation du flux de sang, et les termes « distal » et « proximal » feront référence, respectivement, à un emplacement « amont » et un emplacement « aval ».

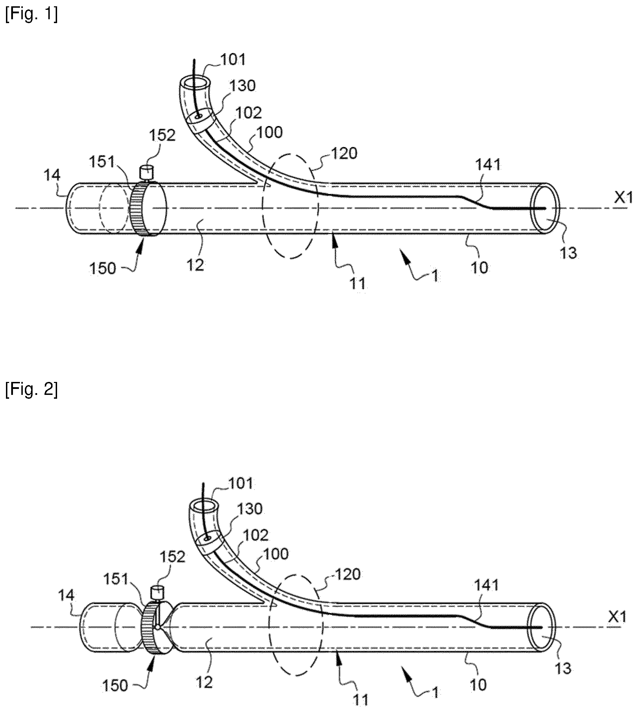

La figure 1 montre une représentation schématique d'un premier mode de réalisation du système selon l'invention.

Le système 1 comprend tout d'abord un premier tube 10 ayant une surface latérale 11, un axe longitudinal X1, un passage intérieur 12 s'étendant le long de l'axe X1, et, à ses extrémités, une ouverture amont 13 et une ouverture aval 14. Le premier tube a par ailleurs un diamètre pouvant prendre les valeurs suivantes : 9 F (3 mm), 10 F (3.33 mm), 12 F (4 mm), 14 F (4.67 mm), 16 F (5.33 mm), ou 18 F (6 mm). L'unité de mesure F est appelée French (« F »), 1 F étant égal à un tiers de millimètre.

Le premier tube 10 est dans un matériau tel que le polytétrafluoroéthylène expansé sous forme microporeuse (ePTFE) ou en polyéthylène téréphtalate sous forme textile (PET). Par exemple, le premier tube 10 peut être en GoreTex(R) ou en Dacron(R).

Le système comprend ensuite un deuxième tube 100 s'étendant entre une entrée 101 du deuxième tube 100 et la surface latérale 11 du premier tube 10. Le deuxième tube 100 communique avec le premier tube 10 à travers la surface latérale 11 du premier tube 10, c'est-à-dire que le passage intérieur 102 du deuxième tube 100 est relié au passage intérieur 12 du premier tube 10. Cette connexion des deux passages intérieur 12 et 102 définit une zone de communication 120 entre le premier tube 10 et le deuxième tube 100. En d'autres termes, le deuxième tube 100 est une dérivation du premier tube 10 au niveau de la surface latérale 11 de ce dernier.

Le deuxième tube 100 a un diamètre inférieur au diamètre du premier tube 10, par exemple le diamètre du deuxième tube est de 2 mm (ou 6F) ou de 2.67 mm (ou 8F).

Le deuxième tube 100 peut être dans le même matériau que le premier tube.

Le deuxième tube 100 comporte une valve 130, insérée dans le passage intérieur 102. La valve 130 comprend dans son volume intérieur un conduit destiné au passage d'un fil-guide support 141 et d'un ensemble de d'instruments interventionnels introduits par l'entrée 101. La valve 130 comprend de plus un dispositif d'étanchéité destiné à empêcher le passage d'un flux de fluide provenant du premier tube 10 et circulant dans le deuxième tube 100.

Le fil-guide support 141 est introduit par l'entrée 101 du deuxième tube 100, puis passé à travers la valve 130 et acheminé jusqu'à l'ouverture amont 13 du premier tube 10 via la zone de communication 120. Au niveau de l'ouverture amont 13, le fil-guide support 141 est de préférence sensiblement centré au centre de l'ouverture amont 13. Le fil-guide support 141, une fois inséré dans le système, n'est plus mobile. Il définit un trajet de guidage pour l'ensemble des instruments interventionnels.

De façon générale, un fil-guide comporte une ame centrale en acier ou en nitinol, des gaines et/ou des spires qui recouvrent l'ame centrale, un revêtement lubrifiant recouvrant les gaines et/ou les spires, et une extrémité distale. Un fil-guide comprend de plus des marqueurs radio-opaques sur l'ame ou l'extrémité. De préférence, le fil-guide support 141 est rigide ou très rigide (« stiff » ou « extra-stiff » en anglais) avec une extrémité distale plus souple, non effilée. Sa longueur est préférence 180 cm et son diamètre 0.889 mm (0.035 pouces) mais d'autres longueurs et diamètres sont possibles, par exemple 300 cm pour la longueur, 0.4572 mm (0.018 pouces) ou 0.3556 (0.014 pouces) pour le diamètre.

Par instrument interventionnel, on entend un dispositif médical utilisé en chirurgie endovasculaire. Un tel dispositif comprend généralement une structure tubulaire avec ou sans lumière, de diamètre généralement compris entre 0.2 mm (0.008 pouces) et 3.33 mm (10 F), et de grande longueur, par exemple de longueur supérieure à 40 cm.

Par ensemble d'instruments interventionnels, on entend un ensemble de dispositifs médicaux utiles pour réaliser une anastomose dans un vaisseau ou un organe. Un tel ensemble comprend un instrument de franchissement, appelé aussi guide de franchissement, et un instrument de maintien, appelé aussi guide de maintien.

Le guide de franchissement est par exemple un fil-guide de franchissement 140, de préférence souple et présentant une extrémité distale effilée (« floppy » et « tappered » en anglais) configurée pour percer la paroi d'un vaisseau ou d'un organe.

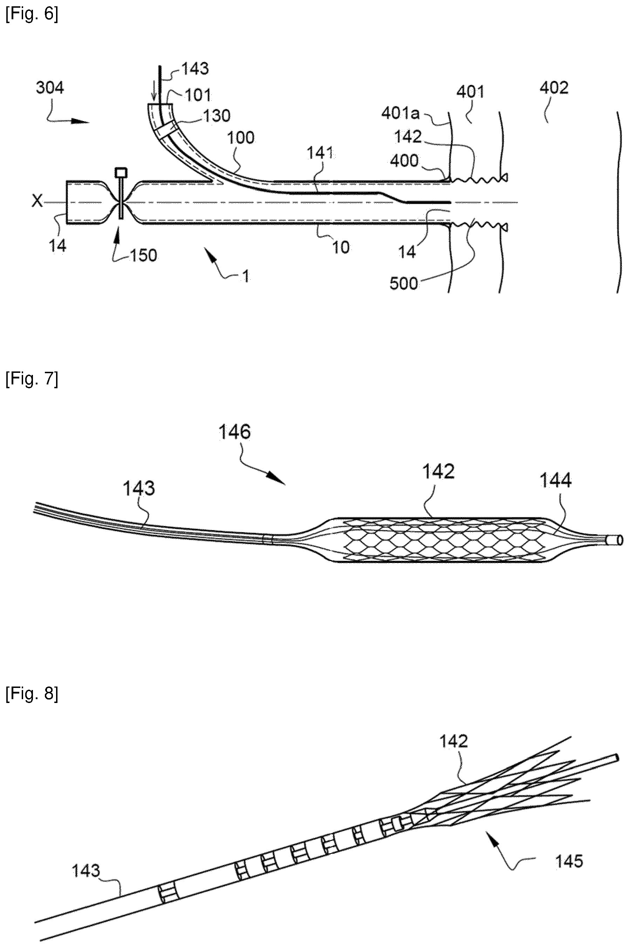

Le guide de maintien est par exemple un cathéter à stent sur ballon 146 tel qu'illustré sur la figure 6. Un tel guide comporte un stent 142 préchargé sur un système d'introduction 143 coopérant avec le fil-guide support 141.

Le stent 142 est comparable à un ressort de forme tubulaire ouverte à ses extrémités, pouvant être étiré lorsqu'une pression/tension lui est appliquée. On entend par « forme déployée » la forme que prend le stent 142 une fois étiré. De préférence, le diamètre du stent, c'est-à-dire le diamètre du stent dans sa forme déployée, est supérieur de 1 mm au diamètre choisi pour le premier tube 10. Par exemple, si le premier tube 10 a un diamètre égal à 10F (3.33 mm) alors le stent aura un diamètre de 4.33 mm.

Dans cette forme déployée, le stent 142 est un tube qui prolonge le premier tube 10 à partir de l'ouverture amont 13, c'est-à dire qui prolonge le premier tube 10 tout en étant adjacent à l'ouverture amont 13.

Le stent 142 est un stent couvert, c'est-à-dire que sa surface ne laisse pas passer le sang.

Le stent 142 est réalisé en métal découpé au laser ou réalisé à partir de modules pré-soudés. Le métal est de préférence un alliage de chrome-cobalt mais peut être aussi en acier ou un alliage de nickel-titane. Le stent 142 a ainsi une bonne visibilité au contrôle scopique (rayons X), une bonne résistance à la déformation, une haute force radiale et une bonne précision à l'étirement.

Le stent 142 est préchargé à l'extrémité distale d'un introducteur 143 tel qu'un cathéter. Un cathéter est une structure tubulaire s'insérant sur le fil-guide support 141. La structure tubulaire et le fil-guide support 141 présentent ainsi un arrangement coaxial : le fil-guide support 141, fixe, agit comme un guide, ou un rail, pour guider le cathéter 143 qui est mobile le long du fil-guide support 142.

Le stent 142 est de plus étiré à l'aide d'un système de largage. En référence à la figure 7 illustrant une portion distale d'un guide de maintien 146, ce système de largage est par exemple un ballonnet 144 monté à l'intérieur du stent et relié par une petite tubulure intégrée à l'introducteur (non représentée), la petite tubulure étant reliée à l'opposé du ballonnet à une insuflateuse (non représentée). Le ballonnet 144 est gonflé pour étirer le stent 142, le laissant dans sa forme déployée. Le ballonnet 144 est ensuite dégonflé et retiré avec l'introducteur 143. Un autre système de largage peut être un stent auto-expansible 145 comme illustré figure 8.

Chaque instrument interventionnel est introduit par l'entrée 101 du deuxième tube 100 et acheminé à travers le système en suivant le trajet de guidage défini par le fil-guide support 141.

Les instruments interventionnels sont mobiles à travers le système. Autrement dit, ils peuvent coulisser dans le système sous l'action du praticien. Les instruments peuvent aussi s'étendre au-delà de l'ouverture 13 dans la direction axiale, c'est-à-dire le long de l'axe longitudinal X1 du premier tube 10.

De préférence, un seul instrument interventionnel est utilisé à la fois. Ainsi, le cathéter 143 est coulissé sur le guide de maintien 146, puis le guide de maintien 146 est retiré et le stent 142 est laissé dans sa forme déployée après retrait de l'introducteur 143 et du ballonnet 144.

Le système 1 comprend de plus un dispositif de ligature 150 agencé sur la circonférence du premier tube 10 en aval de la zone de communication entre le premier et le deuxième tube 120. Ce dispositif de ligature comprend un élément de serrage 151 coopérant avec un mécanisme de serrage 152 permettant de serrer ou de relâcher l'élément de ligature. L'élément de serrage est un clip de ligature 151 disposé autour du premier tube 10 et le mécanisme de serrage est une vis de ligature 152 agissant sur le clip de ligature. Via le mécanisme de serrage, le dispositif de ligature prend deux agencements : un premier agencement serré, illustré figure 2, qui ligature le premier tube à 100%, le fermant ainsi au niveau du clip, ou un deuxième agencement relâché qui laisse le premier tube 10 ouvert (Figure 1).

Décrivons maintenant le système 1 d'un point de vue fonctionnel.

L'anastomose est réalisée au niveau de l'ouverture amont 13 du premier tube 10 du système 1, celle-ci étant au contact de la paroi d'un vaisseau ou d'un organe.

Le premier tube 10 constitue ainsi la prothèse, c'est-à-dire le substitut synthétique/artificiel qui est inséré dans le vaisseau ou l'organe et qui sert de conduit ou d'extension à ce vaisseau ou à cet organe.

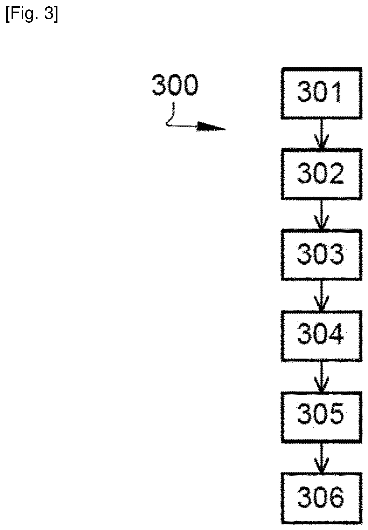

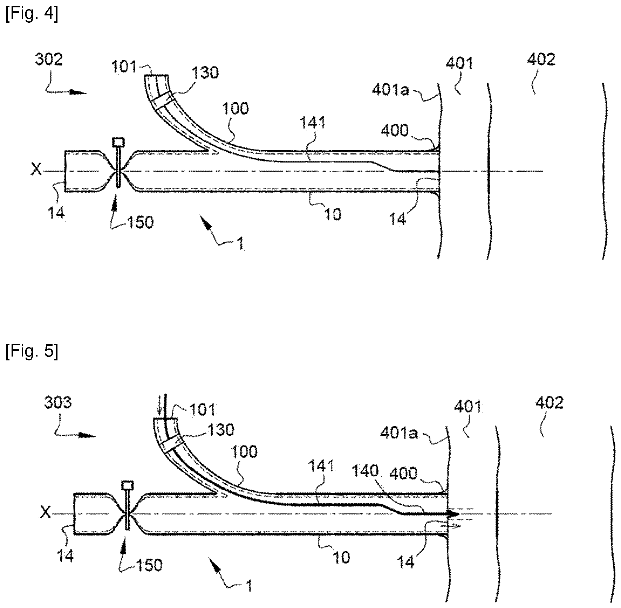

Le deuxième tube 100 sert à acheminer les instruments interventionnels, ceux-ci permettant de réaliser un procédé d'anastomose 300 comportant les étapes d'anastomose 301 à 306 illustrées sur le diagramme de la figure 3. Les étapes 302, 303, 304 et 306 sont en particulier illustrées sur les figures 4,5, 6 et 9. Dans ces figures, le système est anastomosé dans un vaisseau, matérialisé par sa paroi 401 et son volume intérieur 402.

Ces étapes concernent le positionnement du système 1, le percement de la paroi du vaisseau ou de l'organe, l'élargissement du percement et le maintien du système 1 en communication avec le vaisseau ou l'organe. L'étape de maintien comprend le maintien de la dimension de l'ouverture dans la paroi, le maintien de l'étanchéité de la connexion et le maintien des deux structures (vaisseau ou organe et système 1) ensemble.

Dans une première étape 301, le système 1 est acheminé par abord chirurgical sur la paroi périphérique 401a d'un vaisseau ou un organe. Le fil-guide support 141 permet de positionner précisément, par contrôle scopique, le système 1 sur le site d'anastomose. Pour cela, le fil-guide support 141 est préalablement introduit par l'entrée 101 du deuxième tube 100 et acheminé à travers le système 1 jusqu'à être en contact avec la paroi périphérique 401a du vaisseau ou de l'organe.

Dans une deuxième étape 302 illustrée en figure 4, le premier tube 10 est suturé au fil 400 sur la paroi 401 du vaisseau 402. Plus précisément, le premier tube 10 est suturé sur sa circonférence au niveau de l'ouverture amont 13. Ainsi, une première étanchéité est obtenue entre le système 1 et le vaisseau sans qu'aucune ouverture n'ait été faite dans la paroi 401 dudit vaisseau.

Dans une troisième étape 303 illustrée en figure 5, le fil-guide de franchissement 140 est introduit dans le système par l'entrée 101 du deuxième tube 100 et acheminé le long du fil-guide support 141 sur la paroi 401a du vaisseau par l'ouverture amont 13 du premier tube 10. Par l'action d'une vis (non représentée) et grâce à l'extrémité effilée du fil-guide de franchissement 140, une ouverture de diamètre pouvant être inférieur à 0.3 mm est pratiquée à travers la paroi 401 du vaisseau. Du sang provenant du vaisseau est alors acheminé dans le premier tube 10 et le deuxième tube 100. Néanmoins, grâce à la valve 120, le sang ne reflue pas hors du deuxième tube 100, et grâce au dispositif de ligature 140 préalablement agencé dans le premier agencement en position de serrage, le sang ne sort pas non plus hors du premier tube 10. Ainsi, la valve 120 et le dispositif de serrage 140 permettent d'éviter les pertes de sang sans avoir recours à un arrêt de la circulation du sang dans le vaisseau ou l'organe, en amont du site de l'anastomose.

Dans une quatrième étape 304, le fil-guide de franchissement est retiré du système 1 par l'entrée 101 du deuxième tube 100, et le stent 142 est déployé dans l'ouverture de la paroi grâce au guidage par le fil-guide support 141. Plus précisément, en référence à la figure 6, le stent 142 est déployé à la jointure 500 entre le premier tube 10 et le vaisseau ou l'organe, dans la paroi 401. Le stent 142 ainsi déployé impacte les fragments de la paroi 401 du vaisseau ou de l'organe en laissant une lumière circulante cylindrique sans turbulence, donc non thrombogène. Par ailleurs, il maintient après extraction de l'introducteur 143 un calibre d'ouverture à une ouverture prédéterminée, égale au diamètre du premier tube 10 plus 1 mm. En d'autres termes, le stent 142 forme un conduit étanche entre le vaisseau ou l'organe et le système 1, ce conduit ayant de plus une bonne stabilité et un bon maintien, malgré des mouvements liés au corps humain, ou à la manipulation du système 1.

Le stent 142 permet ainsi de réduire encore le risque de fuite de sang. Par ailleurs, il rend l'anastomose rapide, précise, fiable, et reproductible.

Le stent 142 permet de réaliser une endo-anastomose dans le vaisseau. En effet, la connexion est réalisée depuis l'intérieur du vaisseau 402.

Dans une cinquième étape 305, l'ouverture aval 14 du premier tube 10 est anastomosée selon le même procédé que les étapes 301 à 304. Les deux ouvertures amont et aval 13 et 14 du premier tube 10 sont donc anastomosées chacune dans un vaisseau 402, 502. L'ouverture aval du premier tube 14 peut, aussi, être raccordée à un système circulatoire, par exemple une pompe à sang.

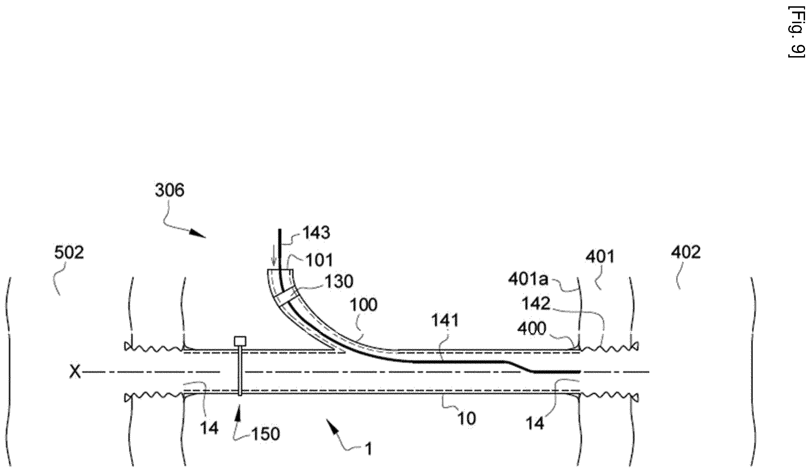

Dans une sixième étape 306 illustrée figure 9, le dispositif de ligature est dans son deuxième agencement, de sorte que le sang circule librement dans le premier tube 10.

Les étapes 301 à 306 sont rapides à réaliser, de l'ordre de quelques minutes. Ainsi, le système 1 évite le recours au clampage.

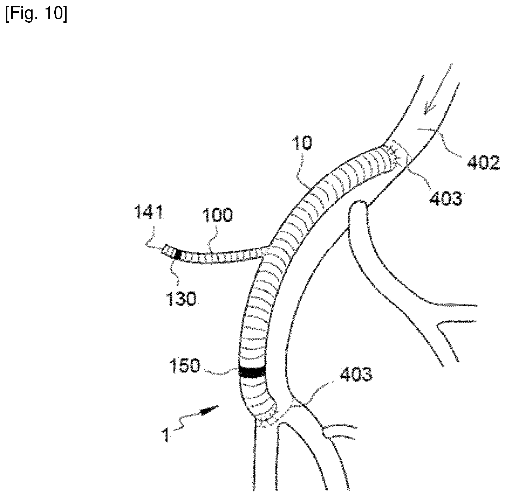

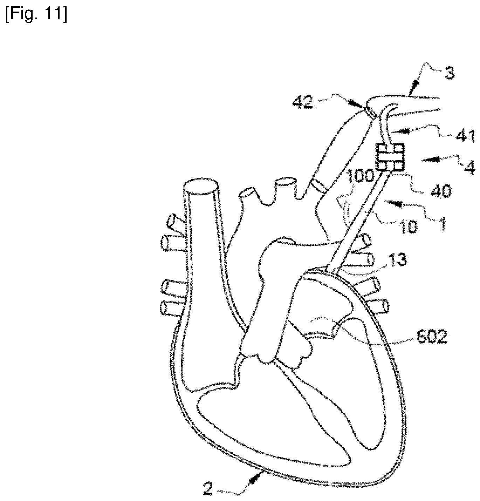

Les figures 10 et 11 illustrent deux applications du système d'anastomose 1.

En référence à la figure 10, la première application consiste en un pontage, par exemple un pontage de l'aorte 402, dans le but de substituer un segment pathologique 403 par le premier tube 10 du système 1. Le segment pathologique 403 est par exemple un anévrisme de l'aorte.

En référence à la figure 11, la deuxième application consiste en l'anastomose de l'oreillette gauche 602 du cœur pour l'amélioration d'un système de pompe à sang de déchargement 4 destiné à traiter les insuffisances cardiaques. Le système 1 est alors utilisé en remplacement d'une canule d'aspiration, ce qui permet de rendre l'accès au cœur beaucoup moins dangereux.

Plus précisément, le système de pompe à sang de déchargement 4 est monté pour décharger du sang de l'oreillette gauche 602 à l'artère sous-clavière gauche 3.

Le système de pompe à sang de déchargement 4 comprend en plus du système 1, une canule de réinjection 41 et une pompe à sang de déchargement 40 et dans cet exemple un organe de ligature en l'occurrence un deuxième clip 42 qui peut être remplacé par un lasso. La pompe à sang 40 comprend un orifice d'entrée 43, raccordé à l'ouverture aval 14 (référencée par exemple sur la figure 1) du système 1 et un orifice de sortie 44 raccordé à une extrémité d'entrée 45 de la canule de réinjection 41. L'ouverture amont 13 du système 1 est située dans l'oreillette gauche 602 et la canule de réinjection 43 comprend une extrémité de reflux 46 opposée à l'extrémité d'entrée 45 située dans cet exemple dans l'artère sous-clavière gauche 3. Le deuxième clip 42 est clipsé sur l'artère sous clavière gauche en amont de l'extrémité de reflux 46, pour ligaturer l'artère. Le deuxième clip 42 ou le lasso peut être adapté à ligaturer entre 85% et 100% une artère sous clavière gauche.

La pompe à sang de déchargement 40 permet donc d'aspirer du sang dans l'oreillette gauche 602 par l'ouverture amont 13 du système 1 et de le rejeter dans l'artère sous clavière 3 par l'extrémité de reflux 46 de la canule de réinjection 41.

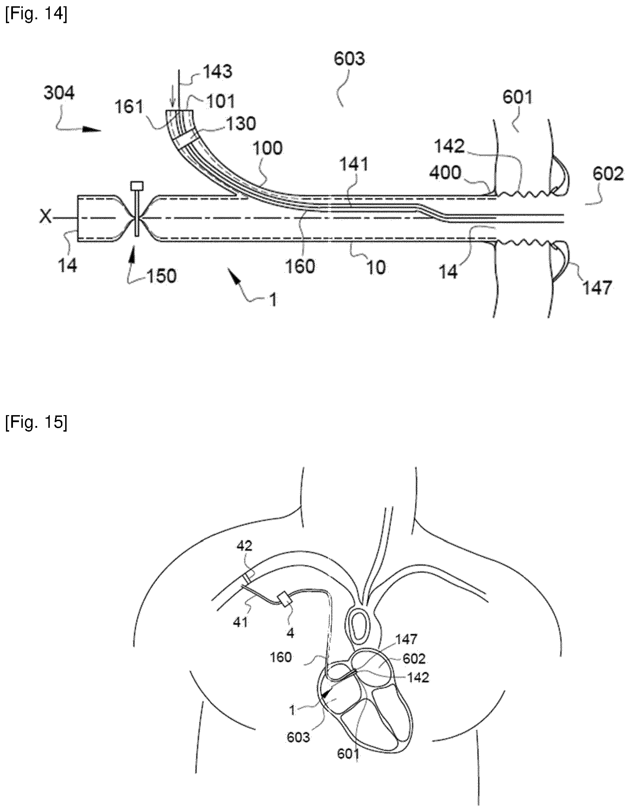

Dans un développement du premier mode de réalisation illustré figure 14, le système 1 comprend un stent 142 comportant un système d'accroche 147, et un troisième tube 160, dit « endo-tube » car inséré à l'intérieur du système 1. Ce troisième tube 160 est introduit par l'entrée 101 du deuxième tube 100 et acheminé jusqu'à l'intérieur du vaisseau ou de l'organe en suivant le trajet de guidage défini par le fil-guide support 141.

Dans ce développement, le diamètre du premier tube (10) est de préférence 6F (2 mm), 8F (2.67 mm), 10 F (3.33 mm), 12 F (4 mm), 14 F (4.67 mm), 16 F (5.33 mm), le diamètre du deuxième tube est de préférence 6F ou 8F, et le diamètre du stent est de préférence supérieur de 1 mm au diamètre du premier tube (10).

Ce développement trouve par ailleurs une application particulièrement avantageuse sur le septum inter-atrial 601, qui est la paroi séparant l'oreillette droite 601 et l'oreillette gauche 601 du cœur.

La figure 15 illustre le système 1 utilisée dans une telle application. Le troisième tube 160 permet de faire circuler le sang provenant de l'oreillette gauche 602 jusque dans une pompe à sang de déchargement 4 via l'oreillette droite 603. Le système d'accroche 147 permet de fournir un point d'accroche du système 1 sur la paroi du septum inter-atrial 601 et d'améliorer la stabilité du maintien même en présence des mouvements cardiaques.

Grâce à l'invention, la pompe à sang de déchargement peut être introduite dans le corps du patient sous la clavicule. Cet abord est beaucoup moins invasif que la thoracotomie requise pour implanter la pompe à déchargement directement dans l'oreillette gauche du cœur.

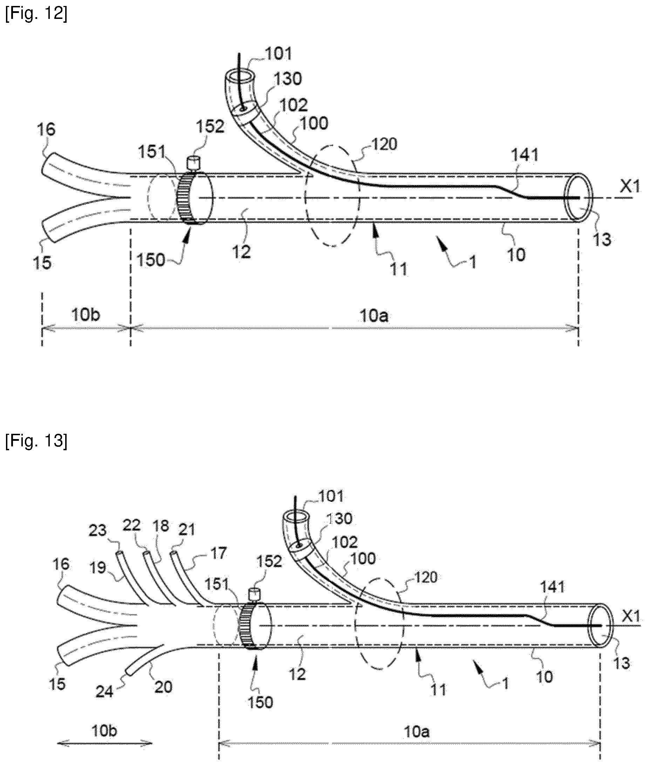

Dans un deuxième mode de réalisation illustré figure 12, le premier tube 10 comprend une partie droite 10a et une partie bifurquée 10b, la bifurcation étant agencée en aval du dispositif de serrage 150. Autrement dit, le premier tube 10 se divise, au niveau de la partie bifurquée 10b, en deux tubes 15, 16 de diamètres égaux, le diamètre étant alors égal à la moitié du diamètre du premier tube 10 sur sa partie droite 10a.

Dans un développement de ce deuxième mode de réalisation illustré figure 13, le premier tube comprend de plus quatre branches latérales 17, 18, 19, 20, sous la forme de quatre tubes de diamètre 6F (2 mm) ou 8F (2.67 mm) s'étirant depuis la surface latérale 11 jusqu'à des entrées de branche 21, 22, 23, et 24. Les branches sont espacées les unes des autres sur la surface latérale 11 du premier tube 10 dans la direction axiale ou circonférentielle. De préférence, trois branches 17, 18 et 19 sont espacées selon la direction axiale, et une branche 20 est espacée sur la circonférence de sorte à être à l'opposé des trois branches.

Ce deuxième mode de réalisation trouve une application pour l'anastomose de l'aorte lors d'une pathologie supra-rénale. En effet, cette configuration permet avantageusement d'anastomoser rapidement les entrées des branches latérales avec les quatre artères principales (artères rénales et artères mésentériques) qui alimentent les reins et le foie. Le système 1 évite le clampage ou réduit celui-ci à quelques minutes (i.e. environ 5 minutes), rendant l'intervention plus légère et, de fait, applicable à des patients âgés ou fragiles.

Quel que soit le mode de réalisation, le système d'anastomose 1 est implanté dans le patient selon une voie dite « hybride » s'appuyant sur les bases de la chirurgie conventionnelle et y associant les avantages d'une endo-anastomose sans clampage. En d'autres termes, le système 1 permet, sans clampage, de respecter au maximum la paroi des vaisseaux tout en assurant une meilleure étanchéité au niveau des extrémités de la prothèse. Le système 1 vise à obtenir des interventions chirurgicales plus légères adaptées à des patients fragiles, et également plus sûres, entrainant de meilleurs résultats peropératoires et, à plus long terme, des durées de séjour en soins intensifs plus courtes, une réduction de la douleur, un rétablissement plus rapide par rapport aux systèmes d'anastomose de l'état de l'art.

## Revendications

1. Système d'anastomose (1) **caractérisé en ce qu'**il comporte :
- un premier tube (10) présentant un axe longitudinal (X1), une surface latérale (11), une ouverture amont (13) et une ouverture aval (14) configurées pour être suturées sur la paroi d'un organe, tel qu'un vaisseau ;
- au moins un deuxième tube (10) s'étendant depuis la surface latérale (11) du premier tube (10) jusqu'à une entrée (101) du deuxième tube (100) et communiquant avec le premier tube (10) en traversant de façon étanche la surface latérale (11), la zone de la surface latérale (11) traversée par le deuxième tube (100) définissant une zone de communication entre le premier et le deuxième tube (120) ;
- une valve (130) montée dans le deuxième tube (100) ; et
- un dispositif de ligature (150) agencé sur la circonférence du premier tube (10) en aval de la zone de communication (120) entre le premier tube (10) et le deuxième tube (100), le dispositif de ligature (150) présentant un premier agencement ou un deuxième agencement, le premier agencement fermant le premier tube (10) et le deuxième agencement laissant ledit premier tube (10) ouvert, dans lequel dans lequel le dispositif de ligature (150) est un clip de ligature (151) muni d'une vis de ligature (152).

2. Système (1) selon la revendication 1 **caractérisé en ce qu'**il comprend un fil-guide support (141) s'étendant de l'entrée (101) du deuxième tube (10) à l'ouverture amont (13) du premier tube (10) en passant par la valve (130), le fil-guide support (141) définissant un trajet de guidage.

3. Système (1) selon la revendication 2 **caractérisé en ce qu'**il comprend en outre au moins un instrument interventionnel choisi parmi un ensemble d'instruments interventionnels comprenant un guide de franchissement (140) adapté pour percer la paroi d'un vaisseau ou d'un organe et un guide de maintien (146) adapté pour réaliser la jonction entre le premier tube (10) et le volume du vaisseau ou de l'organe, chaque instrument interventionnel étant configuré pour suivre le trajet de guidage.

4. Système (1) selon la revendication 3 dans lequel le guide de maintien (146) coopère avec le fil-guide support (141) et comprend un stent (142), le stent (142) étant couvert et présentant une forme déployée de diamètre supérieur au diamètre de premier tube (10), la forme déployée prolongeant le premier tube (10) à partir de l'extrémité amont (13) du premier tube.

5. Système (1) selon la revendication 4 dans lequel le stent (142) est un stent couvert sur ballon.

6. Système (1) selon la revendication 4 ou 5 **caractérisé en ce qu'**un troisième tube (160) traverse le système (1) en suivant le trajet de guidage, et **en ce que** le stent (142) comprend en plus un système d'accroche (147).

7. Système (1) selon la revendication 6 dans lequel le système d'accroche (147) est un autre stent.

8. Système (1) selon l'une quelconque des revendications 1 à 7 dans lequel le premier tube (10) comporte une bifurcation en deux tubes (15,16), la bifurcation définissant sur le premier tube (10) une partie droite (10a) et une partie bifurquée (10b), la bifurcation étant agencée en aval du dispositif de ligature (150) de sorte que la partie droite du premier tube (10a) se trouve en amont du système de ligature (150) et la partie bifurquée (10b) se trouve en aval dudit dispositif de ligature (150).

9. Système (1) selon la revendication 8 dans lequel la partie bifurquée (10b) du premier tube (10) communique avec au moins un quatrième tube (17), le quatrième tube (17) s'étendant entre la surface latérale (11) de la partie bifurquée du premier tube (10b) et une entrée du quatrième tube (21).

## Patentansprüche

1. Anastomosesystem (1), **dadurch gekennzeichnet, dass** es umfasst:
- einen ersten Kanal (10) mit einer Längsachse (X1), einer Seitenfläche (11), einer stromaufwärtigen Öffnung (13) und einer stromabwärtigen Öffnung (14), die so gestaltet sind, dass sie an der Wand eines Organs, beispielsweise eines Gefäßes, angenäht werden können;
- mindestens einen zweiten Kanal (100), der sich von der Seitenfläche (11) des ersten Kanals (10) bis zu einem Eingang (101) des zweiten Kanals (100) erstreckt und mit dem ersten Kanal (10) in Verbindung steht, indem er die Seitenfläche (11) abdichtend durchquert, wobei der Bereich der Seitenfläche (11), der vom zweiten Kanal (100) durchquert wird, einen Verbindungsbereich zwischen dem ersten und dem zweiten Kanal (120) bildet;
- ein im zweiten Kanal (100) angebrachtes Ventil (130); und
- eine Ligaturvorrichtung (150), die am Umfang des ersten Kanals (10) stromabwärts des Verbindungsbereichs (120) zwischen dem ersten Kanal (10) und dem zweiten Kanal (100) angeordnet ist, wobei die Ligaturvorrichtung (150) eine erste Anordnung oder eine zweite Anordnung aufweist, wobei die erste Anordnung den ersten Kanal (10) verschließt und die zweite Anordnung den ersten Kanal (10) offen lässt, wobei die Ligaturvorrichtung (150) eine Ligaturklemme (151) ist, die mit einer Ligaturschraube (152) versehen ist.

2. System (1) nach Anspruch 1, **dadurch gekennzeichnet, dass** es einen Stützführungsdraht (141) umfasst, der sich vom Eingang (101) des zweiten Kanals (10) über das Ventil (130) bis zur stromaufwärtigen Öffnung (13) des ersten Kanals (10) erstreckt, wobei der Stützführungsdraht (141) einen Führungsweg definiert.

3. System (1) nach Anspruch 2, **dadurch gekennzeichnet, dass** es außerdem mindestens ein Interventionsinstrument umfasst, das aus einer Reihe von Interventionsinstrumenten ausgewählt ist, darunter eine Überbrückungsführung (140), die zum Durchstechen der Wand eines Gefäßes oder Organs geeignet ist, und eine Halteführung (146), die zum Herstellen der Verbindung zwischen dem ersten Kanal (10) und dem Volumen des Gefäßes oder Organs geeignet ist, wobei jedes Interventionsinstrument so gestaltet ist, dass es dem Führungsweg folgt.

4. System (1) nach Anspruch 3, bei dem die Halteführung (146) mit dem Stützführungsdraht (141) zusammenwirkt und einen Stent (142) umfasst, wobei der Stent (142) überzogen ist und eine entfaltete Form mit einem Durchmesser aufweist, der größer ist als der Durchmesser des ersten Kanals (10), wobei die entfaltete Form den ersten Kanal (10) vom stromaufwärtigen Ende (13) des ersten Kanals aus verlängert.

5. System (1) nach Anspruch 4, bei dem der Stent (142) ein ballonüberzogener Stent ist.

6. System (1) nach Anspruch 4 oder 5, **dadurch gekennzeichnet, dass** ein dritter Kanal (160) das System (1) entlang des Führungswegs durchquert und dass der Stent (142) zusätzlich ein Befestigungssystem (147) umfasst.

7. System (1) nach Anspruch 6, bei dem das Befestigungssystem (147) ein weiterer Stent ist.

8. System (1) nach einem der Ansprüche 1 bis 7, bei dem der erste Kanal (10) eine Verzweigung in zwei Kanäle (15, 16) aufweist, wobei die Verzweigung am ersten Kanal (10) einen geraden Abschnitt (10a) und einen verzweigten Abschnitt (10b) definiert und die Verzweigung stromabwärts der Ligaturvorrichtung (150) angeordnet ist, so dass sich der gerade Abschnitt des ersten Kanals (10a) stromaufwärts der Ligaturvorrichtung (150) und der verzweigte Abschnitt (10b) stromabwärts der Ligaturvorrichtung (150) befindet.

9. System (1) nach Anspruch 8, bei dem der verzweigte Abschnitt (10b) des ersten Kanals (10) mit mindestens einem vierten Kanal (17) in Verbindung steht, wobei sich der vierte Kanal (17) zwischen der Seitenfläche (11) des verzweigten Abschnitts des ersten Kanals (10b) und einem Eingang des vierten Kanals (21) erstreckt.

## Claims

1. An anastomosis system (1) **characterised in that** it includes:
- a first tube (10) having a longitudinal axis (X1), a side surface (11), an upstream opening (13) and a downstream opening (14) configured to be sutured on the wall of an organ such as a vessel;
- at least one second tube (10) extending from the side surface (11) of the first tube (10) to an inlet (101) of the second tube (100) and communicating with the first tube (10) by sealingly passing through the side surface (11), the zone of the side surface (11) through which the second tube (100) passes defining a communication zone between the first and second tubes (120);
- a valve (130) mounted in the second tube (100); and
- a ligation device (150) arranged on the circumference of the first tube (10) downstream of the communication zone (120) between the first tube (10) and the second tube (100), the ligation device (150) having a first arrangement or a second arrangement, the first arrangement closing the first tube (10) and the second arrangement leaving said first tube (10) open, wherein the ligation device (150) is a ligation clip (151) provided with a ligation screw (152).

2. The system (1) according to claim 1, **characterised in that** it comprises a support guide wire (141) extending from the inlet (101) of the second tube (10) to the upstream opening (13) of the first tube (10) by passing through the valve (130), the support guide wire (141) defining a guide path.

3. The system (1) according to claim 2, **characterised in that** it further comprises at least one interventional instrument selected from a set of interventional instruments comprising a crossing guide (140) adapted to pierce the wall of a vessel or organ and a holding guide (146) adapted to make junction between the first tube (10) and the volume of the vessel or organ, each interventional instrument being configured to follow the guide path.

4. The system (1) according to claim 3, wherein the holding guide (146) cooperates with the support guide wire (141) and comprises a stent (142), the stent (142) being covered and having an expanded shape with a diameter greater than the diameter of the first tube (10), the expanded shape extending from the upstream end (13) of the first tube (10).

5. The system (1) according to claim 4, wherein the stent (142) is a covered balloon stent.

6. The system (1) according to claim 4 or 5, **characterised in that** a third tube (160) passes through the system (1) following the guide path, and **in that** the stent (142) additionally comprises an attachment system (147).

7. The system (1) according to claim 6, wherein the attachment system (147) is another stent.

8. The system (1) according to any one of claims 1 to 7, wherein the first tube (10) comprises a bifurcation into two tubes (15,16), the bifurcation defining on the first tube (10) a straight portion (10a) and a bifurcated portion (10b), the bifurcation being arranged downstream of the ligation device (150) so that the straight portion of the first tube (10a) is upstream of the ligation system (150) and the bifurcated portion (10b) is downstream of said ligation device (150).

9. The system (1) according to claim 8, wherein the bifurcated portion (10b) of the first tube (10) communicates with at least one fourth tube (17), the fourth tube (17) extending between the side surface (11) of the bifurcated portion of the first tube (10b) and an inlet of the fourth tube (21).
